(19) Europäisches Patentamt
European Patent Office
Office européen des brevets

(11) EP 1 440 687 A1

(12) EUROPEAN PATENT APPLICATION
published in accordance with Art. 158(3) EPC

(43) Date of publication:
**28.07.2004 Bulletin 2004/31**

(21) Application number: **02777815.8**

(22) Date of filing: **09.10.2002**

(51) Int Cl.7: **A61K 31/09**, A61K 35/78, A61P 39/06, A61P 43/00

(86) International application number:
**PCT/JP2002/010471**

(87) International publication number:
**WO 2003/030888 (17.04.2003 Gazette 2003/16)**

(84) Designated Contracting States:
**AT BE BG CH CY CZ DE DK EE ES FI FR GB GR IE IT LI LU MC NL PT SE SK TR**
Designated Extension States:
**AL LT LV MK RO SI**

(30) Priority: **09.10.2001 JP 2001310960**

(71) Applicant: **The Japanese Research & Development Association for New Functional Foods**
**Nihonbashi, Chuo-Ku, Tokyo 103-0001 (JP)**

(72) Inventors:
- **MORIMURA, Shigeru**
  **Kikuchi-gun, Kumamoto 861-1104 (JP)**
- **KIDA, Kenji**
  **Kumamoto-shi, Kumamoto 862-0924 (JP)**
- **MAEDA, Hiroshi**
  **Kumamoto-shi, Kumamoto 862-0909 (JP)**
- **YASUDA, Fumi; c/o Showa Sangyo Co., Ltd.**
  **Funabashi-shi, Chiba 273-0015 (JP)**
- **WAKAMATSU, Daisuke;**
  **c/o Showa Sangyo Co., Ltd.**
  **Funabashi-shi, Chiba 273-0015 (JP)**
- **TSUNEHIRO, Jun; c/o Showa Sangyo Co., Ltd.**
  **Funabashi-shi, Chiba 273-0015 (JP)**
- **NAKAI, Chiaki; c/o Showa Sangyo Co., Ltd.**
  **Funabashi-shi, Chiba 273-0015 (JP)**

(74) Representative:
**Forstmeyer, Dietmar, Dr. rer. nat., Dipl.-Chem. et al**
**Boeters & Lieck,**
**Bereiteranger 15**
**81541 München (DE)**

# (54) ANTIRADICALS AND FAT COMPOSITIONS, FOODS, DRINKS, DRUGS OR FEEDS CONTAINING THE ANTIRADICALS

(57) It is intended to obtain highly active and fat-soluble antiradicals originating in a natural substance whereby the stability of foods (fat compositions) can be elevated and disorders caused by oxidation *in vivo* can be prevented simultaneously. Namely (1) an antiradical containing 4-vinyl-2,6-dimethoxyphenol as the active ingredient; (2) an antiradical comprising an extract of crude rapeseed oil containing 4-vinyl-2,6-dimethoxyphenol; (3) a crude rapeseed oil extract containing 4-vinyl-2,6-dimethoxyphenol in a concentrated state characterized by being prepared by treating crude rapeseed oil with an alcohol or an aqueous alcohol or distilling it; and (4) an antiradical containing these three types of crude rapeseed oil extracts.



Printed by Jouve, 75001 PARIS (FR)

## Description

### Technical Field of the Invention

The present invention relates to antiradicals, and fat compositions, foods, drinks, drugs or feeds containing the antiradicals.

### Background Art

Radical substances such as active oxygen are known to induce oxidative disorders *in vivo,* which cause various diseases as well as aging. Lipid peroxides (alkyl hydroperoxides) which are generated by oxygen damage of lipids are assumed to accumulate in cell membranes and organs that contain abundant lipid, because they are made of lipids and have long life. In addition, considering that lipid radicals are generated in the intestinal tract, lipid peroxides are also assumed to easily contact the epithelial cells of the intestinal tract to damage them, and to be taken into the lymph system to reach the blood by being converted to chylomicrons.

Other than chylomicrons, various lipid-protein complexes are present in the blood, such as low-density lipoproteins (LDLs) and high-density lipoproteins (HDLs). Lipids in LDLs are subjected to chain-like lipid hyperoxidation due to metal ions or above-mentioned lipid radicals, easily resulting in the formation of oxidized LDLs. The oxidized LDLs are considered to be a significant risk factor of arterio sclerosis.

Thus, to scavenge radicals originating from lipid peroxides is important in the suppression of oxidative damage *in vivo.* There are many studies regarding antioxidative effects, including the following.

1) A water-soluble ingredient extracted from defatted rapeseeds has an antioxidative effect (H. Nowak et al., Fat Sci. Technol. , 94, 149-152 (1992); U. Wanasundara et al., J. Agric. Food Chem. , 42, 1285-1290 (1994)).
2) A water extract from plants such as vegetables has a radical-scavenging and -removal effect (H. Maeda, Environ. Mutagen Res., 18, 53-61 (1996)).
3) Lipid-peroxide suppressors containing a solvent extract from the plant of the family Myrtaceae (JP, A, 10-36278).
4) Free radical scavengers containing a defatted substance extracted from Perilla seeds or germinated materials thereof by water and/or a hydrophilic organic solvent (JP, A, 2000-236847).
5) Antioxidants containing an extract from olive plant as the active ingredient (JP, A, 2001-181632).

All of these contain a water-soluble ingredient as an active ingredient, and thus they are water-soluble antiradicals.

In contrast, the presence of fat-soluble antiradicals is important when utilization in oil and fat products as well as *in-vivo* efficacy are considered. Fat-soluble antiradicals include butylhydroxyanisol (BHA), butylhydroxytoluene (BHT) and the like; however, since they are obtained by chemical synthesis, their use tends to be avoided.

Degummed oil derived from rapeseeds is known as a fat-soluble antiradical derived from a natural substance (JP, A, 9-157687), but its activity is not sufficient.

Tocopherol is known as a fat-soluble antioxidant derived from a natural substance. However, while tocopherol has a certain level of effect in suppressing the oxidation of usual food, its effect as a lipid-radical scavenger is not sufficient.

Therefore, the development of highly active fat-soluble antiradicals derived from natural substances is being awaited.

### Disclosure of the Invention

The present invention aims at obtaining highly active fat-soluble antiradicals derived from natural substances, which are able to increase the stability of foods (fat compositions) and to prevent oxidative damage *in vivo* simultaneously.

As a result of extensive research to solve the above-mentioned problems, the inventors of the present invention found that an alcohol extract from crude rapeseed oil has a high antiradical activity, and completed the invention after further studies.

Namely, the present invention consists of the following items.

1. An antiradical containing 4-vinyl-2,6-dimethoxyphenol as the active ingredient.
2. An antiradical consisting of an extract of crude rapeseed oil containing 4-vinyl-2,6-dimethoxyphenol.
3. A crude rapeseed oil extract containing concentrated 4-vinyl-2,6-dimethoxyphenol, characterized in that the crude rapeseed oil is treated with alcohol or aqueous alcohol.

4. A crude rapeseed oil extract containing concentrated 4-vinyl-2,6-dimethoxyphenol, characterized in that the crude rapeseed oil is distilled.
5. An antiradical containing the crude rapeseed oil extract according to the above items 3 or 4.
6. A fat composition comprising the antiradical according to at least one of the above items 1 to 5.
7. Foods, drinks, drugs or feeds wherein the antiradical according to at least one of the above items 1 to 5, or the fat composition according to the above item 6 is added.

The structural formula of 4-vinyl-2,6-dimethoxyphenol is as follow:

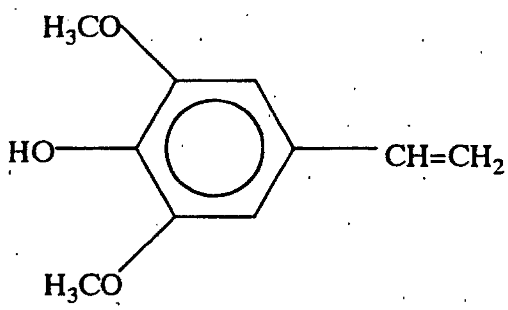

Antiradicals according to the present invention are characterized in that they contain 4-vinyl-2,6-dimethoxyphenol as the active ingredient.
The inventors of the present invention found that an alcohol extract from crude rapeseed oil has a high antiradical activity, and they searched for this active ingredient to discover that the ingredient is 4-vinyl-2,6-dimethoxyphenol; thus they achieved the present invention.
4-vinyl-2,6-dimethoxyphenol, the active ingredient of the antiradicals according to the invention, is advantageous in that it is easily soluble to fat, and has a high antiradical activity.
Antioxidants extracted from conventional defatted rapeseed are hardly fat-soluble. In contrast, the active ingredient of the antiradicals of the present invention is extracted from crude rapeseed oil and is fat-soluble, thus the antiradicals of the invention can be easily used as antioxidants for edible oils and other fat compositions.
When the antiradicals of the present invention are taken into the body, because they have high affinity to the cell membranes *in vivo* due to their fat-soluble properties, they effectively react in the cell membranes and organs that are abundant with fat, where water-soluble antiradicals are hard to react.
Furthermore, the antiradicals of the present invention are advantageous in terms of safety and raw material, because its active ingredient is obtained from an extract of crude rapeseed oil.
In addition to the antiradical properties, the stability of fat compositions in which the antiradicals of the invention are added improves.
The fact that 4-vinyl-2,6-dimethoxyphenol, the active ingredient of the antiradicals of the invention, is easily soluble to fat and has a high antiradical activity, was found for the first time in the present invention; therefore, the choice of said ingredient is of extreme significance.

Brief Description of Drawings

Fig.1: Measurement result of NMR of isolated and purified 4-vinyl-2,6-dimethoxyphenol.
Fig. 2: Measurement results of HPLC of 4-vinyl-2,6-dimethoxyphenol.

Best Mode for carrying out the Invention

The present invention is hereinafter described in detail.

(1) Extraction

4-vinyl-2,6-dimethoxyphenol, the active ingredient of the antiradicals of the present invention, can be concentrated by the extraction from crude rapeseed oil using alcohol.

Rapeseed oil is an oil obtained from rapeseeds which belong to Cruciferae Brassica. Crude rapeseed oil used for the extraction can be selected from expressed oils, extracted oils, degummed oils, etc.

Alcohol used for the extraction includes, methanol, ethanol, propanol, butanol or hydrous substances thereof and the like.

The water content suitable for the extraction differs depending on the kinds of alcohol; it is 0-10% by volume formethanol, and 5-20% by volume for ethanol.

There is no limitation in the extraction conditions. For example, the temperature may be in the range of 10-85°C, and the extraction may be suitably carried out even at room temperature. The use of vibration extraction or an extractor equipped with a stirrer can increase the extraction efficiency. The duration of extraction is from several minutes to several hours. The longer the extraction duration, the larger the amount of extract, however, there is a possibility that the concentration of 4-vinyl-2,6-dimethoxyphenol in the extract becomes low.

The extraction is carried out using alcohol with an amount 0.1-20 folds that of a rawmaterial; preferably, the same extraction procedure is repeated for 2-3 times.

This extraction is a liquid-liquid extraction of fat and alcohol, and an extract containing 4-vinyl-2,6-dimethoxyphenol is obtained by separation and concentration of the alcohol fraction.

Various methods are selected as an extraction method, and attention should be given to the solubility of alcohol and fat. In particular, since ethanol has fairly high solubility to fat, preferably the extraction is carried out using ethanol with an amount 0.1-1 folds that of the raw material, in order to increase the concentration of 4-vinyl-2,6-dimethoxyphenol and to decrease the triglyceride concentration in the extract.

4-vinyl-2,6-dimethoxyphenol, the active ingredient of the antiradicals of the present invention, can also be concentrated by the distillation of crude rapeseed oil.

The distillation may be carried out using any apparatuses, such as a thin-membrane distillation apparatus or centrifugal distillation apparatus. In addition to a low-vacuum distillation apparatus, a high-vacuum distillation apparatus (molecular distillation apparatus) can also be used.

There is no specific limitation in distillation conditions. For example, distillation can be suitably carried out with a temperature of 70-300°C, pressure of 0.1-10 Torr, and duration from several minutes to several hours. While sufficient distillation is performed by higher temperatures, higher levels of vacuum and longer distillation time, there is a possibility of a decrease in the concentration of 4-vinyl-2,6-dimethoxyphenol and a contamination of undesirable ingredients in terms of odor and flavor due to the distillation of other ingredients contained in the crude rapeseed oil. Preferably, the distillation is carried out at 130-180°C, for 10 min- 2 h, and at 1.0-3.0 Torr. Distillation efficiency can be further increased by steam blowing.

If necessary, 4-vinyl-2,6-dimethoxyphenol can also be concentrated by the purification and isolation using liquid-liquid distribution method, column chromatography, thin-layer chromatography (TLC), high-performance liquid chromatography (HPLC) and the like, depending on conditions.

It is sufficient that the amount of 4-vinyl-2,6-dimethoxyphenol in the antiradicals is 0.2% by weight or more.

Accordingly, methanol- or ethanol-extracts, distillates and the like containing 0.2% by weight or more of 4-vinyl-2,6-dimethoxyphenol can be used as antiradicals as they are.

(2) Application

The antiradicals according to the invention may be taken into the body as they are; in addition, they may be mixed with fat compositions such as common edible refined oils to obtain antiradical fat compositions, and these compositions may be added to foods and drinks to produce antiradical foods and drinks.

In products added with the antiradicals according to the invention (fat compositions, etc.), not only their antiradical properties, but also their stability is improved.

As foods and drinks, for example snacks (chewing gum, candy, caramel, chocolate, cookie, munch, jelly, gummi, and TABLET, etc.), noodles (backwheat noodle, Japanese wheat noodle, Chinese noodle, etc.), dairy products (milk, ice-cream, yogurt, etc.), drinks (juice, coffee, tea, green tea, carbonated drink, sport drink, etc.) are included.

Additives such as anti-degradation agents, sweeteners, flavoring agents, colors, emulsifying agents, antioxidants and fillers may be appropriately added to foods and drinks.

Similarly, the antiradicals according to the invention and antiradical fat compositions can be added to drugs, cosmetics, feeds, etc. to obtain antiradical drugs, cosmetics, feeds, etc.

The addition of 10-5000 ppm of 4-vinyl-2,6-dimethoxyphenol to fat compositions produces antiradical fat compositions, which can fully demonstrate their effects.

Hereinafter, the present invention is further described using working examples; however, the invention is not limited to these examples. Here, "%" means "% by weight" unless specified otherwise.

(1) Isolation and purification of 4-vinyl-2,6-dimethoxyphenol

4-vinyl-2,6-dimethoxyphenol was isolated and purified from crude rapeseed oil by the following 5 steps.

1. Methanol extraction

Methanol 90 ml was added to 450 g of crude rapeseed oil, and the mixture was vigorously stirred for 10 min using a shaker. After the stirring, the mixture was centrifuged at 5000 rpm for 30 min. The upper methanol layer was collected in an eggplant-type flask. Again, methanol 90 ml was added to the lower-layer crude rapeseed oil, and the mixture was stirred using a shaker. This procedure was repeated for a total of three times. The methanol was evaporated fromthemethanol layer collected, and 2.9 g of an extract was obtained.

2. Liquid-liquid distribution

The 2.9-g methanol extract obtained by the step 1 was subjected to the liquid-liquid distribution using the same method described in Example 5, and 1.5 g of a hexane fraction and 0.86 g of an acetonitrile fraction were obtained.
Since a high antiradical activity was observed in the acetonitrile fraction, this fraction was used in the next purification step.

3. Fractionation by silica gel column chromatography

15 g of silica gel (Kanto Kagaku, for column chromatography, < 150 μm) was filled into a glass column of 300 mm x 20 mmϕ. The 500-mg acetonitrile fraction obtained by the step 2 was dissolved in 5 ml of chloroform, and then introduced into the column. Isolation solvents (hexane/diethylether (90/10) 175 ml, chloroform 225 ml, acetone 400 ml and methanol 200 ml, with this sequence) were supplied into the column at a rate of approximately 2 ml/min for elution. The eluted fractions were collected and stored in an eggplant-type flask, and the solvents were evaporated to obtain 144 mg of a chloroform fraction, 205 mg of an acetone fraction and 42 mg of a methanol fraction.
Since the highest activity was observed in the chloroform fraction, this fraction was used in the next step.

4. Preparative isolation by thin-layer chromatography (TLC)

The 100-mg chloroform fraction obtained by the step 3 was subjected to the development using a TLC plate (silica gel: Merk Kieselgel 60)/developing solvent (hexane/diethylether = 80/30). Upon confirmation of a yellow band on the plate, the objective substance together with the silica gel was scraped by a spatula. Chloroform 20-30 ml was added, and after shaking it up well, silic gel was filtered off using a glass filter. Then, the solvent was evaporated to obtain 13 mg of a concentrate. The obtained concentrate was used in the next step.

5. Preparative isolation by high-performance liquid chromatography (HPLC)

2 mg of the concentrate obtained by the step 4 was subjected to the elution by HPLC under the following conditions. The eluent A (100%) was flown for the 0-20th min, then the eluent A (100%) was changed to the eluent B (100%) from the 20th to 25th min with a linear gradient, and the eluent B (100%) was flown from the 25th min and after. The single peak observed at around the 4.5th min was isolated. The yield was 1 mg.
(Elution conditions for HPLC)
Column: Keystone Scientific, Inc. AQUASIL C18 (4.6 I.D. x 250 mm)
Amount of sample: 100 μl (5 mg/ml)
Eluent A: $CH_3CN/H_2O$ (90/10)
Eluent B: $CH_3CN$
Column temperature: room temperature
Flow rate: 1.0 ml/min
Detector: UV detector (300 nm, 450 nm)

(2) Identification of 4-vinyl-2,6-dimethoxyphenol

The following analysis method was used for the identification.

1) LC/MS

(Analysis method)

A sample was dissolved into methanol, and the measurement was performed with APCI (+) using LC/MS (JEOL, JMS-LCmate).

(Analysis result)

m/z of the major ingredient measured by the low-resolution MS was 181.
According to the measurement result of molecular weight by the above low-resolution MS, the molecular weight of the m/z 181 ingredient is 180, because ionization easily occurs at M+H with APCI(+).
Therefore, the accurate value of the molecular weight of the m/z 181 ingredient was measured by the high-resolution (milli-mass) MS, and it was determined to be 181.0813.
Results of the CHN analysis and S analysis conf irmed the absence of N and S.
Based on these results, the molecular formula of the m/z 181 ingredient is $C_{10}H_{13}O_3$; thus the calculated value of the molecular weight is 181.0865. Since there is almost no difference in the molecular weight between the calculated value and the above measurement value, the molecular formula of the major ingredient was determined to be $C_{10}H_{12}O_3$.

2) NMR

(Analysis method)

The sample was dried under a nitrogen flow, dissolved in chloroform-$d_1$ ($CDCl_3$), and introduced into a special glass tube; then NMR (JEOL, 400 MHz) was measured.

(Analysis result)

Figure 1 shows analysis result of NMR.
From this result, the following findings can be derived.
3.90 ppm: six H atoms of $-OCH_3$ x 2 (line symmetry)
5.17 ppm: an H atom at the trans position of C=C
5.51 ppm: an H atom of the phenol
5.62 ppm: an H atom at the cis position of C=C
6.61 ppm: an H atom at the gem position of C=C and two H atoms in the benzene ring
In addition, the absence of a peak at 1-3 ppm revealed the absence of an alkyl group (C-C).
The above results show that -OH and $-OCH_3$ x 2 (line symmetry) are bound to the benzene ring, and H is bound to C=C, while an alkyl group (C-C) is absent.
Considering this analysis result with reference to the calculated value of the chemical shift, the purified substance was identified to be 4-vinyl-2,6-dimethoxyphenol.

(3) Quantification of 4-vinyl-2,6-dimethoxyphenol

The HPLC method described in the above (1)-5. was repeated for several times, and a standard sample of 4-vinyl-2,6-dimethoxyphenol for quantification was obtained.
Using this standard sample, a calibration curve was produced by HPLC analysis shown hereinafter; then concentrations of 4-vinyl-2,6-dimethoxyphenol in each sample were determined.
(conditions of HPLC analysis)
Column: LiChroCART 250 - 4 LiChrospher 100 $NH_2$ (4.0ID x 244 mm, 5 µm)
Eluent: 1.2% isopropyl alcohol/hexane (v/v)
Flow rate: 1.2 ml/min
Column temperature: Room temperature (25°C or lower)
Detector: Fluorescence detector (Ex 298 nm, Em 325 nm)
Amount of the sample: 50 µl
Internal standard reagent: pentamethyl chromanol (PMC)
Measurement time: 30 min
Figure 2 shows the results of the analysis. PMC was detected at around 6 min (peak 3), and 4-viny 1-2,6-dimethoxyphenol was detected at around 18 min (peak 7).

(4) Antiradical activity

Antiradical activities were measured using the following method.

1) $IPOX_{50}$ value

A concentration of a test substance at which 50% of the chemiluminescence is suppressed is measured by a chemiluminescence method, us ing a measurement system with tertial butylhydroperoxide (t-BuOOH) as a model compound of fat peroxides (alkyl peroxides: ROOH).

50 μl of 10-mM diethylenetriamine pentaacetic acid (Sigma Chemical, Co.), 50 μl of 300-mM t-BuOOH (Sigma Chemical Co.), 50 μl of 100-mM luminol (Wako Junyaku, Co., Ltd.), and 50 μl of ethanol solution of a test substance are added to 250 μl of 0.01-M phosphate buffered saline, and the mixture is stirred well, then 50 μl of 1-mg/l hemoglobin (from pig: Sigma Chemical Co.) is added and the mixture is immediately set on an extremely-weak-luminescence measuring device (Berthold LB9505); then the amount of luminescence is measured. The concentration of the test substance at which 50% of the luminescence is suppressed relative to the case without addition of the test-substance solution is measured, and this value is designated to be 50% inhibitory potential of peroxyl radicals concentration ($IPOX_{50}$). It is meant that smaller the value of $IPOX_{50}$, the larger the radical scavenger capacity.

2) Trolox relative activity in ORAC method

10 μl of a test substance in a designated concentration, or 10 μl of a 60-μM 6-hydroxy-2,5,7,8-tetramethylchroman-2-carboxylic acid (Trolox) solution is added to 10 μl of 200-fold diluted solution of human blood serum. Then, 180 μl of 3.73-mg/l β-phycoerythrin (β-PE) is added, and the mixture is preheated at 37°C for 1 min. 10 μl of 80-mM 2,2'-azobis(2-amidinopropane)-dihydrochloride (AAPH) is then added, and the reaction is started at 37°C. For the dilution of the serum or reagents added, phosphate buffer (75 mM, pH 7.0) is used. As a control, this phosphate buffer is used in place of the test substance. Fluorescence intensity (Ex: 540 nm, Em: 565 nm) of the reactant is measured every 2 min after the start of the reaction, and the relationship between the fluorescence intensity (y-axis) and the time (x-axis) is plotted on a graph. The area (S) enclosed by the obtained curve, x and y axes is calculated for the test substance, Trolox and the control. The antiradical activity of the test substance in the serum is calculated as a Trolox relative activity using the following equation.

$$\text{Trolox relative activity} = (\text{Test substance's S} - \text{Control's S})/(\text{Trolox's S} - \text{Control's S}) \times (\text{Trolox's concentration}/\text{Test substance's concentration})$$

In this testing system, AAPH generates peroxyradicals, and the peroxyradicals oxidize the substrate β-PE, which results in a reaction to eliminate fluorescence of β-PE. Trolox is a derivative of water-soluble vitamin E, and is used as a standard substance of the antioxidation reaction in this testing system. These reaction substrate and standard substance are those used in an oxygen radical absorbance capacity (ORAC) method. The ORAC method is widely used to obtain antioxydation and antiradical activities of biological samples and natural materials as values relative to those of a standard substance, in an easy and accurate manner.

(5) Rancimat test

This test is to measure oxidative stability of oils and fats. In this test, while a sample is heated to 120°C in a reaction vessel, clean air is flown into the container, and volatile decomposed matters generated by the oxidation are collected under water, and the duration until the flexure point when electric conductivity of the water rapidly changes (Rancimat time) is measured.

(6) Stability test during frying

The stability test during frying was carried out by following method.

500 g of a test oil is measured in a pot (diameter: 23 cm), and heated to 180°C by an electric heater. After

achieving a specified temperature, a potato piece (diameter, 4 cm; thickness, 0.6 cm) is immediately deep-fried for 3 min. Potato pieces are deep-fried every 1 hour until 5 hours while maintaining the temperature at 180°C. Foam at 20 s after the introduction of the potato pieces into the oil are observed and their amounts are assessed by a ratio of the foam area to the entire surface area of the oil in 5 scales (A: 0-20%, B: 21-40%, C: 41-60%, D: 61-80%, E: 81-100%).

Example 1, Comparative Example 1

60-μM 4-vinyl-2,6-dimethoxyphenol and 300-μM α-tocopherol were tested to calculate Trolox relative activities, as listed in Table 1.

Table 1

| | Test substance | Trolox relative activity |
|---|---|---|
| Example 1 | 4-vinyl-2,6-dimethoxyphenol | 0.58 |
| Comparative Example 1 | α-tocopherol | 0.08 |

As shown in Table 1, the Trolox relative activity of 4-vinyl-2,6-dimethoxyphenol in serum was approximately 7 folds that of α-tocopherol. This indicates that 4-vinyl-2,6-dimethoxyphenol in serum expresses extremely stronger antiradical activity than α-tocopherol.

Example 2 (Ethanol extract)

200 ml of 99.5% ethanol was added to 1 kg of crude rapeseed oil and the mixture was vigorously shaken for 10 min. The ethanol layer was separated by centrifugation. This process was repeated three times. The collected ethanol layers were concentrated to remove the ethanol, and 21 g of an extract was obtained.

Example 3 (Aqueous ethanol extract)

200 ml of 90% aqueous ethanol was added to 1 kg of crude rapeseed oil and the mixture was vigorously shaken for 10 min. The ethanol layer was separated by centrifugation. This process was repeated three times. The collected ethanol layers were concentrated to remove the ethanol and water, and 8 g of an extract was obtained.

Example 4 (Methanol extract)

200 ml of 99.5% methanol was added to 1 kg of crude rapeseed oil and the mixture was vigorously shaken for 10 min. The methanol layer was separated by centrifugation. This process was repeated three times. The collected methanol layers were concentrated to remove the methanol, and 7 g of an extract was obtained.

Example 5 (2-layer distribution acetonitrile extract)

The 7-g methanol extract obtained in Example 4 was dissolved in 45 ml of hexane, and the mixture was introduced into a separating funnel 1. 15 ml of 99.5% acetonitrile was added to the separating funnel 1, and the funnel was shaken. After the shaking, the lower layer (acetonitrile layer) was transferred into separating funnel 2 containing 45 ml of new hexane, while 15 ml of new acetonitrile was introduced into the separating funnel 1. The separating funnels 1 and 2 were shaken, then the lower layer of the separating funnel 2 was introduced into an eggplant-type flask, and the lower layer of the separating funnel 1 was introduced into the separating funnel 2. Another 15 ml of acetonitrile was introduced into the separating funnel 1, then the similar procedure was repeated for a total of two times. The lower layers separated were combined and concentrated to remove the acetonitrile, and 1.5 g of an extract was obtained.

Example 6 (Distillate)

1000 g of crude rapeseed oil was introduced into a Claisen flask, and the molecular distillation was carried out at 150°C for 30 min, under 1.5 Torr with 1.5% of steam blowing amount vs. oil. Water was evaporated from matters collected from the trap, and 3 g of an distillate was obtained.

Example 7 (4-vinyl-2,6-dimethoxyphenol)

In this experiment, 4-vinyl-2,6-dimethoxyphenol isolated and purified from crude rapeseed oil as in the above (3) was used.
Table 2 lists antiradical activities and contents of 4-vinyl-2,6-dimethoxyphenol of the extracts, distillate and 4-vinyl-2,6-dimethoxyphenol obtained by the above examples.
For comparison, Table 2 also lists antiradical activities of crude rapeseed oil (Comparative Example 2), 100% vitamin E (Comparative Example 3), 5% vitamin E (Comparative Example 4), 1% vitamin E (Comparative Example 5), which were measured similarly.
In Comparative Examples 3, 4 and 5, vitamin E with α-Toc 68.1%, β-Toc 0.4% and δ-Toc 0.1% was used and antiradical activities were measured by dissolving the vitamin E into rapeseed salad oil.

Table 2.

| | Ingredient | $IPOX_{50}$ (mg/ml) | 4-vinyl-2,6-dimethoxyphenol (%) |
|---|---|---|---|
| Example 2 | Ethanol extract | $9.5 \times 10^{-2}$ | 0.9 |
| Example 3 | Aqueous ethanol extract | $9.2 \times 10^{-3}$ | 2.1 |
| Example 4 | Methanol extract | $8.5 \times 10^{-3}$ | 2.3 |
| Example 5 | 2-Layer distribution acetonitrile extract | $5.0 \times 10^{-4}$ | 9.2 |
| Example 6 | Distillate | $6.5 \times 10^{-4}$ | 7.1 |
| Example 7 | 4-Vinyl-2,6-dimethoxyphenol | $1.0 \times 10^{-4}$ | 100 |
| Comparative Example 2 | Crude rapeseed oil | 1.9 | 0.022 |
| Comparative Example 3 | 100% vitamin E | $5.5 \times 10^{-2}$ | - |
| Comparative Example 4 | 5% vitamin E | 15 | - |
| Comparative Example 5 | 1% vitamin E | 86 | - |

The Results listed in Table 2 show that 4-vinyl-2,6-dimethoxyphenol or substances containing it have superior antiradical activities than conventional substances.

Example 8

The ethanol extract obtained in Example 2 was added to refined rapeseed oil at a ratio of 2% to obtain an antiradical refined rapeseed oil.

Example 9

The methanol extract obtained in Example 4 was added to refined rapeseed oil at a ratio of 0.7% to obtain an antiradical refined rapeseed oil.

Example 10

The acetonitrile fraction obtained in Example 5 was added to refined rapeseed oil at a ratio of 0.15% to obtain an antiradical refined rapeseed oil.

Example 11

The distillate obtained in Example 6 was added to refined rapeseed oil at a ratio of 0.2% to obtain an antiradical refined rapeseed oil.
Table 3 lists antiradical activity and stability of the antiradical refined rapeseed oils obtained in Examples 8-11.

Table 3.

| | | Refined rapeseed oil without additives | Example 8 | Example 9 | Example 10 | Example 11 |
|---|---|---|---|---|---|---|
| $IPOX_{50}$ (mg/ml) | | >100 | 5.9 | 5.9 | 0.53 | 5.4 |
| Rancimat time (h) | | 3.9 | 5.0 | 5.6 | 4.3 | 5.4 |
| Stability test during frying | 0h | A | A | A | A | A |
| | 1h | A | A | A | A | A |
| | 2h | A | A | A | A | A |
| | 3h | B | A | A | A | A |
| | 4h | B | A | A | A | A |
| | 5h | D | A | A | B | A |
| Increase of acid value in 5-h | | 0.31 | 0.11 | 0.12 | 0.19 | 0.25 |

The results shown in Table 3 indicate that the antiradical activities and stabilities during frying, etc. of the fat compositions added with the antiradicals of the present invention increase.

Industrial Applicability

(1) The antiradicals of the present invention contain 4-vinyl-2,6-dimethoxyphenol as the active ingredient, and are superior in that they have high antiradical activities.
(2) Antioxidants extracted from conventional defatted rapeseed meal are hardly fat-soluble. In contrast, the active ingredient of the antiradicals of the present invention is extracted from crude rapeseed oil and is fat-soluble, and thus can be easily used as antioxidants for edible oils and other fat compositions.
(3) When the antiradicals of the present invention are taken into the body, because they have high affinity to the cell membranes *in vivo* due to their fat-soluble properties, they effectively react in the cell membranes and organs that are abundant with fat, where water-soluble antiradicals are hard to react.
(4) The antiradicals of the present invention are advantageous in terms of safety and raw material, because its active ingredient is obtained from an extract of crude rapeseed oil.
(5) Products (fat compositions, etc.) added with the antiradicals of the present invention gain not only an antiradical properties, but also improved stability.

## Claims

**1.** An antiradical containing 4-vinyl-2,6-dimethoxyphenol as the active ingredient.

**2.** An antiradical consisting of an extract of crude rapeseed oil containing 4-vinyl-2,6-dimethoxyphenol.

**3.** A crude rapeseed oil extract wherein 4-vinyl-2,6-dimethoxyphenol is concentrated, **characterized in that** the crude rapeseed oil is treated with alcohol or aqueous alcohol.

**4.** A crude rapeseed oil extract wherein 4-vinyl-2,6-dimethoxyphenol is concentrated, **characterized in that** the crude rapeseed oil is distilled.

**5.** An antiradical containing the crude rapeseed oil extract according to claims 3 or 4.

**6.** A fat composition comprising the antiradical according to any of claims 1 to 5.

**7.** Foods, drinks, drugs or feeds wherein the antiradical according to any of claims 1 to 5, or the fat composition according to claim 6 is added.

FIG. 1

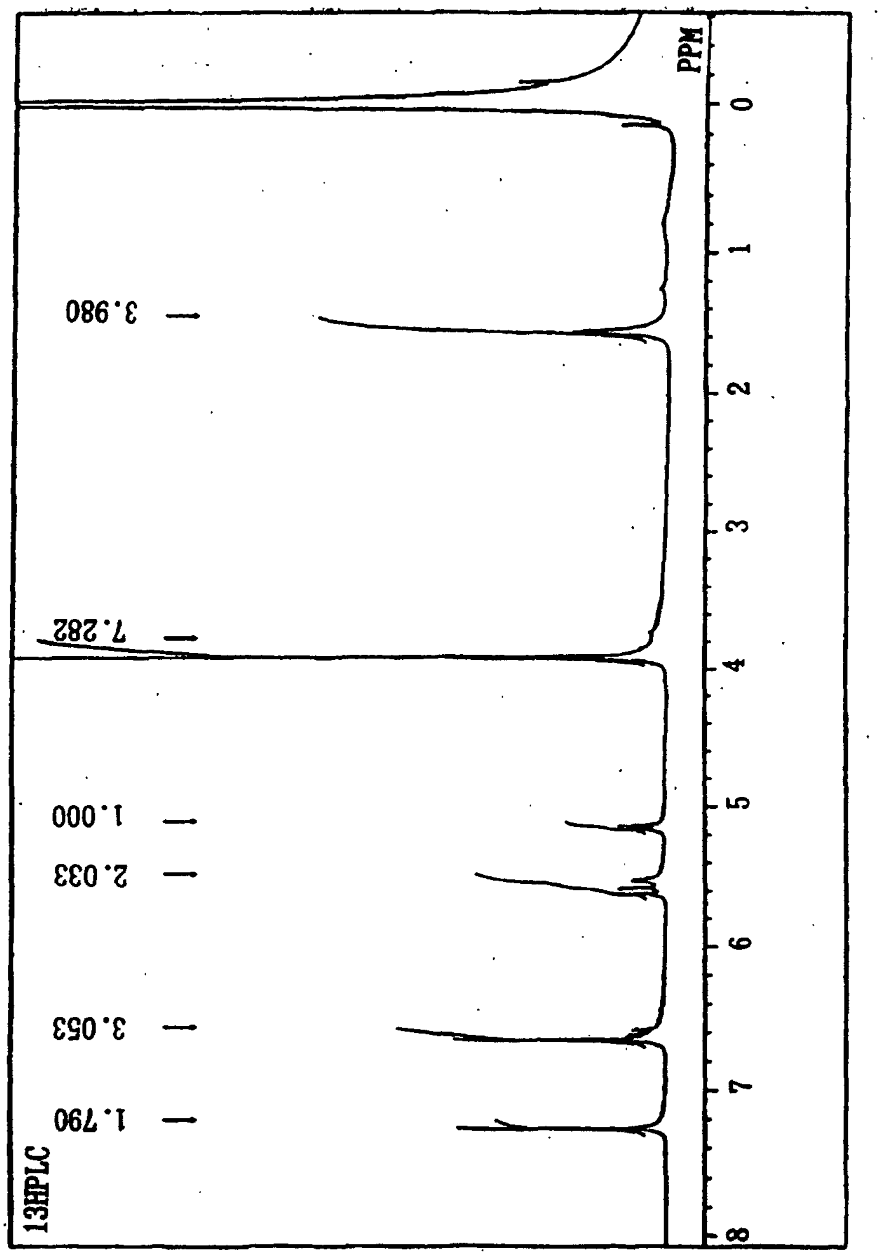

FIG.2

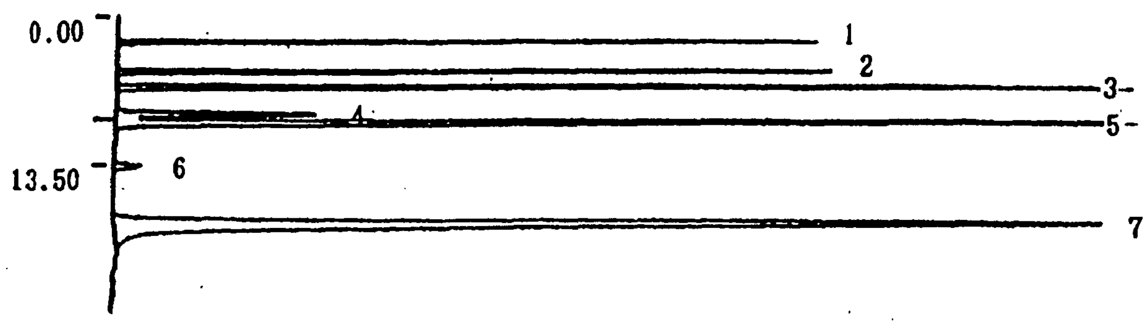

## INTERNATIONAL SEARCH REPORT

International application No.
PCT/JP02/10471

A. CLASSIFICATION OF SUBJECT MATTER
Int.Cl[7] A61K31/09, 35/78, A61P39/06, 43/00

According to International Patent Classification (IPC) or to both national classification and IPC

B. FIELDS SEARCHED

Minimum documentation searched (classification system followed by classification symbols)
Int.Cl[7] A61K31/09, 35/78

Documentation searched other than minimum documentation to the extent that such documents are included in the fields searched

Electronic data base consulted during the international search (name of data base and, where practicable, search terms used)
CA(STN), REGISTRY(STN), WPIDS(STN)

C. DOCUMENTS CONSIDERED TO BE RELEVANT

| Category* | Citation of document, with indication, where appropriate, of the relevant passages | Relevant to claim No. |
|---|---|---|
| X<br>A | JP 9-157687 A (Showa Sangyo Co., Ltd.), 17 June, 1997 (17.06.97), Full text (Family: none) | 2,4-7<br>1,3 |
| X<br>A | Espin, Juan Carlos; Soler-Rivas, Cristina; Wichers, Harry J., Characterization of the total free radical scavenger capacity of vegetable oils and oil fractions Using 2,2-Diphenyl-1-picrylhydrazyl radical, Journal of Agricultural and Food Chemistry (2000), 48(3), 648-656 | 2-7<br>1 |
| X<br>A | Nowak, H.; Kujawa, K.; Zadernowski, R.; Roczniak, B.; Kozlowska, Halina, Antioxidative and bactericidal properties of phenolic compounds in rapeseeds, Fett Wissenschaft Technologie (1992), 94(4), 149-52 | 2-7<br>1 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 07 November, 2002 (07.11.02) | 19 November, 2002 (19.11.02) |
| Name and mailing address of the ISA/<br>Japanese Patent Office | Authorized officer |
| Facsimile No. | Telephone No. |

Form PCT/ISA/210 (second sheet) (July 1998)